# FASCICULE DE BREVET EUROPEEN

(11) **EP 2 688 597 B1**
(45) Date de publication et mention de la délivrance du brevet: **11.05.2016**
(21) Numéro de dépôt: 12714822.9
(22) Date de dépôt: 21.03.2012
(51) Int. Cl.: A61L 9/12

(54) **DISPOSITIF ET SYSTEME DE DIFFUSION DE PARFUM**
VORRICHTUNG UND SYSTEM ZUR VERBREITUNG VON DUFTSTOFFEN
DEVICE AND SYSTEM FOR DIFFUSION OF FRAGRANCE

(30) Priorité: 22.03.2011 FR 1152360
(43) Date de publication de la demande: 29.01.2014
(73) Titulaire: Presensia, 75002 Paris (FR)
(72) Inventeur: SUISSA, David, F-94300 Vincennes (FR); WETS, Frédéric, F-75020 Paris (FR)
(74) Mandataire: IP Trust
(86) Numéro de dépôt international: PCT/FR2012/050588
(87) Numéro de publication internationale: WO 2012/131232

(56) Documents cités:
- FR-A1- 2 877 845
- US-A- 3 795 438
- US-A- 4 059 422
- US-A1- 2005 082 695
- US-A1- 2006 219 962
- US-A1- 2008 193 328
- US-A1- 2009 151 447

## Description

L'invention concerne de manière générale le domaine de la diffusion de parfum.

L'invention concerne plus particulièrement un dispositif compact de diffusion de parfum.

On entend ici par dispositif 'compact', un dispositif peu encombrant et léger, de sorte d'être préhensible d'une main. A titre d'exemple, le présent dispositif compact peut être déplacé aisément par un unique utilisateur d'une pièce à une autre d'un appartement à parfumer, ou d'un emplacement dans une pièce à un autre emplacement dans la même pièce. Notamment, la compacité du présent dispositif oppose ce dernier à tout dispositif encombrant et lourd dont l'installation fait parfois partie intégrante de l'immobilier ou du mobilier.

Divers dispositifs compacts de diffusion de parfum(s) sont connus de l'état de l'art.

Le plus souvent, ces dispositifs compacts comprennent un réservoir de substances à l'état liquide que l'on vaporise par le biais d'une micronisation du liquide via une buse en exploitant l'effet Venturi. Dans certains dispositifs sophistiqués, la vaporisation est facilitée par l'ajout de vibrations acoustiques à hautes fréquences ayant pour effet de diminuer la taille des gouttes vaporisées, donc d'augmenter la surface du liquide en contact avec l'air, et ainsi améliorer la diffusion du parfum.

Dans certains autres dispositifs, les molécules de parfum(s) sont préalablement intégrées dans des supports solides, par exemple des polymères. La diffusion des substances dans l'air s'effectue en chauffant ces supports solides et/ou au moyen d'un flux d'air léchant la surface de ces supports solides, techniques qui ont pour effet de détacher progressivement les molécules odorantes des supports solides et de les diffuser dans l'air ambiant.

Dans le domaine de la diffusion de parfums, on distingue les diffuseurs adaptés pour diffuser un seul parfum et les diffuseurs adaptés pour diffuser plusieurs parfums différents. On parle de diffuseurs mono parfum et de diffuseurs multi parfums.

Les diffuseurs multi parfums sont conçus le plus souvent pour diffuser un seul parfum à la fois. La sélection du parfum diffusé est issue d'un choix de l'utilisateur ou est commandée par des moyens informatiques ou télévisuels dans le cadre d'oeuvres multimédia multi sensorielles. Ces diffuseurs multi parfums sont relativement complexes à concevoir et sont relativement coûteux.

Un exemple de diffuseur multi parfums est décrit dans le document US-A-3795438.

Les diffuseurs multi parfums sont confrontés à la difficulté d'éviter la pollution entre les parfums due aux migrations de molécules olfactives entre les réservoirs de parfums différents, ou due à la contamination ou imprégnation des éléments du dispositif par les différentes molécules olfactives. La difficulté provient du fait que tout parfum, même sec, c'est-à-dire intégré sur un support solide, est composé de molécules qui s'incrustent ou s'imprègnent dans les matériaux qui constituent le dispositif. Ces matériaux peuvent dès lors être qualifiés de réactifs aux parfums ; tel est, par exemple, le cas de la plupart des plastiques. Or il est connu que le mélange de plusieurs parfums est incompatible avec la subtilité des odeurs recherchée.

Par imprégnation des molécules de parfum dans les éléments constitutifs d'un dispositif, on entend ici l'action par laquelle les molécules de parfum parvenues en contact avec lesdits éléments y pénètrent et s'y répandent de façon diffuse.

Le document FR02620622A1 propose une solution au problème de la pollution entre parfums dans un diffuseur multi parfums. Ce document décrit une cartouche structurée en plusieurs zones absorbantes que l'on imbibe d'une substance liquide, les zones étant séparées entre elles par des éléments non adsorbants pour éviter la migration des molécules de parfums différents entre zones. Ce document ne traite pas de la pollution entre les zones imbibées et le reste du dispositif diffuseur.

Dans le cas des diffuseurs mono parfum, la migration ou l'imprégnation des molécules de parfums vers ou par les constituants du dispositif, respectivement, n'induit pas un problème de pollution entre parfums dès lors que ceux-ci diffusent toujours le même parfum, ce qui est le cas le plus fréquent. Une fois vide, le diffuseur est soit jeté soit rechargé avec la même substance liquide ou des supports solides imprégnés de la même substance.

Le problème lié à la pollution entre les parfums se pose pour les diffuseurs mono parfum conçus pour diffuser séquentiellement plusieurs parfums différents, au choix de l'utilisateur, car les molécules olfactives d'un premier parfum migrent du réservoir de substance liquide ou des supports solides imprégnés vers les différents éléments du dispositif et s'y incrustent. En conséquence, lorsque l'utilisateur substitue un second parfum au premier, le second parfum va être pollué par le reliquat du premier.

Une solution à ce problème est décrite dans le document EP1054697 dans lequel la substance volatile est contenue dans un réservoir dont l'une des faces est de forme sphérique afin de permettre son isolation au moyen d'une rotation du réservoir lorsque l'on ne souhaite pas diffuser d'odeur. Ce dispositif présente les inconvénients suivants : la dimension du dispositif est obligatoirement importante eu égard celle de la partie olfactive active puisque cette dernière est contenue dans une petite partie d'un canal cylindrique pratiqué dans un élément de forme orbiculaire ; la forme orbiculaire et la fonction d'étanchéité de la surface sphérique sont relativement complexes à produire. Au final, le dispositif décrit dans ce document est très complexe, peu compact et relativement coûteux.

Il est encore connu un diffuseur mono parfum développé par la société ScentAir et commercialisé sous l'appellation commerciale ScentWave®. Ce dispositif comprend un couloir de ventilation, un ventilateur et un réservoir de parfum. Généralement, les ventilateurs équipant ce type de dispositif sont des ventilateurs bon marché qui comportent des pâles en matériau plastique dans lesquelles les molécules de parfum s'incrustent aisément. Pour autant, le problème de la pollution entre parfums diffusés consécutivement ne se pose pas pour ce dispositif du fait que le couloir de ventilation, le ventilateur et le réservoir de parfum forment un ensemble solidaire, ledit ensemble étant substitué à un autre pour changer le parfum diffusé ou recharger le dispositif en parfum. On comprend que ledit ensemble constitue une recharge complexe à concevoir et relativement coûteuse.

Dans ce contexte, la présente invention propose un dispositif compact de diffusion de parfum permettant de pallier un ou plusieurs des inconvénients précédemment évoqués. La présente invention propose plus particulièrement un dispositif compact de diffusion de parfum tel que défini dans la revendication 1, qui permette de résoudre le problème de la pollution entre parfums, qui soit simple à utiliser et de faible coût.

A cette fin, le dispositif de l'invention est essentiellement tel qu'il comprend au moins :
- un couloir de ventilation comprenant une entrée et une sortie, et
- un générateur de flux d'air agencé pour propulser l'air dans le couloir de ventilation, de sorte que l'air circule d'amont en aval depuis l'entrée jusqu'à la sortie du couloir de ventilation,
- un support de fixation agencé en aval du générateur de flux d'air et adapté pour y fixer une cartouche de parfum, de sorte que la cartouche de parfum se trouve sur le passage de l'air propulsé,
- une cloison située dans le couloir de ventilation entre le générateur de flux d'air et le support de fixation, l'état de la cloison étant susceptible d'être modifié entre au moins :
   ∘ un état de cloisonnement dans lequel la cloison empêche l'air de circuler entre un premier espace du couloir de ventilation en amont de la cloison et un second espace du couloir de ventilation en aval de la cloison, et
   o un état de non-cloisonnement dans lequel la cloison laisse l'air circuler entre les premier et second espaces du couloir de ventilation, et
- un asservissement entre le générateur de flux d'air et la cloison adapté pour que la cloison soit en état de cloisonnement lorsque le générateur de flux d'air est à l'arrêt,
la cloison et le couloir de ventilation étant réalisés au moins en partie en un matériau inerte aux parfums qui ne s'imprègne pas de parfums.

Tout en présentant une simplicité d'utilisation et un faible coût (au moins du fait que le générateur de flux d'air et le couloir de ventilation ne sont pas des consommables au même titre que la cartouche de parfum) qui en font un produit grand public, le dispositif permet ainsi avantageusement de résoudre le problème de la pollution entre parfums en empêchant la diffusion des molécules de parfum(s) depuis la cartouche de parfum jusque dans ledit premier espace du couloir de ventilation dans lequel se situe le générateur de flux d'air, et ce que le dispositif soit en fonctionnement ou non.

Selon une particularité, les parties du couloir de ventilation et de la cloison réalisées en un matériau inerte aux parfums sont constituées des surfaces internes limitant le second espace du couloir de ventilation en aval de la cloison dans son état de cloisonnement.

Le dispositif présente ainsi avantageusement un coût faible dans la mesure où les parties réalisées en un matériau inerte aux parfums, généralement plus coûteux que le plastique, sont restreintes au strict minimum.

Selon une autre particularité non conforme à l'invention, le dispositif comprend un capteur agencé pour détecter au moins que la cloison quitte l'état de cloisonnement, l'asservissement consistant en une électronique de contrôle propre à mettre en fonctionnement le générateur de flux d'air lorsque le capteur détecte que la cloison quitte l'état de cloisonnement.

Le dispositif prévoit ainsi avantageusement la mise en fonctionnement automatique du générateur de flux d'air dès lors que la cloison n'est plus en son état de cloisonnement.

Le capteur est en outre agencé pour détecter que la cloison atteint l'état de cloisonnement, l'électronique de contrôle étant en outre propre à arrêter le générateur de flux d'air lorsque le capteur détecte que la cloison atteint l'état de cloisonnement.

Le dispositif présente ainsi avantageusement une simplicité et un confort d'utilisation accrus et permet automatiquement de ne pas laisser inutilement le générateur de flux d'air en fonctionnement lorsque la cloison est en son état de cloisonnement.

Selon l'invention le dispositif comprend en outre un interrupteur propre à mettre en fonctionnement et à arrêter le générateur de flux d'air, l'asservissement consistant en une électronique de contrôle propre à modifier l'état de la cloison de sorte que :
- la cloison quitte l'état de cloisonnement après que le générateur de flux d'air ait été mis en fonctionnement, et
- la cloison atteint l'état de cloisonnement avant que le générateur de flux d'air soit à l'arrêt.

Le dispositif permet ainsi une gestion plus fine du fonctionnement du dispositif en ce sens que la diffusion de parfum dans le premier espace comportant le générateur de flux d'air est évitée que le générateur de flux d'air soit en fonctionnement, à l'arrêt ou dans un régime transitoire.

Selon un premier mode de réalisation, la cloison comprend un panneau agencé pour coopérer avec une fente traversante pratiquée dans le couloir de ventilation, de sorte que le panneau coulisse dans la fente.

Le dispositif prévoit ainsi avantageusement un mode de réalisation comportant une cloison de conception simple et peu onéreuse.

Selon une première variante du premier mode de réalisation, le coulissement du panneau dans la fente est réalisé par renversement du dispositif, le panneau coulissant sous l'action de son propre poids.

Le dispositif présente ainsi avantageusement un agencement simple et de faible coût du panneau avec le reste du dispositif.

Selon une seconde variante du premier mode de réalisation, le dispositif comprend une vis sans fin agencée pour faire coulisser le panneau dans la fente, la vis sans fin étant entraînée en rotation par un moteur, le panneau coulissant verticalement d'une position basse correspondant à l'état de cloisonnement à une position haute correspondant à l'état de non-cloisonnement, une tension minimale aux bornes du moteur permettant le maintien du panneau en position haute, et, l'ensemble formé du moteur et de la vis sans fin étant adapté pour laisser retomber le panneau lorsque la tension minimale devient inférieure à un certain seuil.

Le dispositif prévoit ainsi avantageusement qu'en cas de panne d'alimentation en énergie du générateur de flux d'air ou en cas d'énergie faible, le panneau retombe en son état de cloisonnement.

Selon un deuxième mode de réalisation, la cloison comprend un diaphragme.

Le dispositif permet ainsi avantageusement de faire varier le débit d'air atteignant la cartouche de parfum.

Selon un troisième mode de réalisation, la cloison comprend une persienne qui comprend une pluralité de lames. Chaque lame est montée mobile en rotation autour d'un axe horizontal de façon à se soulever sous la force de l'air propulsé par le générateur de flux d'air et à s'affaisser lorsque le générateur de flux d'air s'arrête.

Le dispositif permet ainsi avantageusement d'asservir la modification de l'état de cloisonnement de la cloison au fonctionnement du générateur de flux d'air uniquement de façon mécanique, ou plus particulièrement sans requérir une électronique de contrôle.

Selon une autre particularité, le dispositif comprend une seconde cloison agencée en aval du support de fixation.

Le dispositif permet ainsi avantageusement d'éviter complètement une diffusion naturelle des molécules de parfum même si le générateur de flux d'air n'est pas utilisé.

Selon une autre particularité, le dispositif comprend des moyens d'insertion et de retrait d'au moins une cartouche dans le couloir de ventilation au niveau du support de fixation.

Selon une autre particularité, le générateur de flux d'air comprend une turbine générant une surpression de l'air traversant la cartouche qui est comprise entre 25 et 200 pascals.

Selon une autre particularité, le dispositif se présente sous la forme générale d'un parallélépipède de :
- moins de 20 cm de large, préférentiellement 5,3 cm de large,
- plus de 2 cm et moins de 20 cm de profondeur, préférentiellement inférieure à 5 cm, et
moins de 20 cm de haut, préférentiellement 11,5 cm de haut.

Selon une autre particularité, le dispositif comprend en outre un dispositif de détection de l'absence de cartouche dans le couloir de ventilation, ce dispositif conjointement avec l'électronique de contrôle permettant d'éviter la mise en fonctionnement du générateur de flux d'air en cas d'absence d'au moins une cartouche dans le couloir de ventilation.

Selon une autre particularité, le dispositif comprend en outre une source de lumière et un capteur d'intensité lumineuse situés de part et d'autre du support de fixation et agencés de sorte de capter un état d'usure de la cartouche et en avertir l'utilisateur.

L'invention concerne également un système de diffusion de parfum essentiellement tel qu'il comprend le dispositif selon l'invention et une cartouche de parfum adaptée pour être fixée dans le support de fixation.

Le système présente ainsi avantageusement un faible coût, au moins du fait que le générateur de flux d'air et le couloir de ventilation ne sont pas des consommables au même titre que la cartouche de parfum, cette dernière étant le seul élément du système à devoir être remplacé pour changer le parfum diffusé ou recharger le dispositif en parfum.

D'autres caractéristiques et avantages de l'invention ressortiront clairement de la description qui en est faite ci-après, à titre indicatif et nullement limitatif, en référence aux dessins annexés, dans lesquels :
- la figure 1 représente schématiquement le dispositif selon l'invention,
- les figures 2a et 2b représentent le système selon l'invention dans le premier mode de réalisation du dispositif selon l'invention,
- la figure 3 représente la cloison selon le deuxième mode de réalisation du dispositif selon l'invention,
- la figure 4 représente le système selon l'invention dans le troisième mode de réalisation du dispositif selon l'invention,
- la figure 5 représente une partie du dispositif selon une variante de son premier mode de réalisation, et
- la figure 6 illustre le principe de fonctionnement du dispositif dans son mode de réalisation apte à avertir de l'usure de la cartouche.

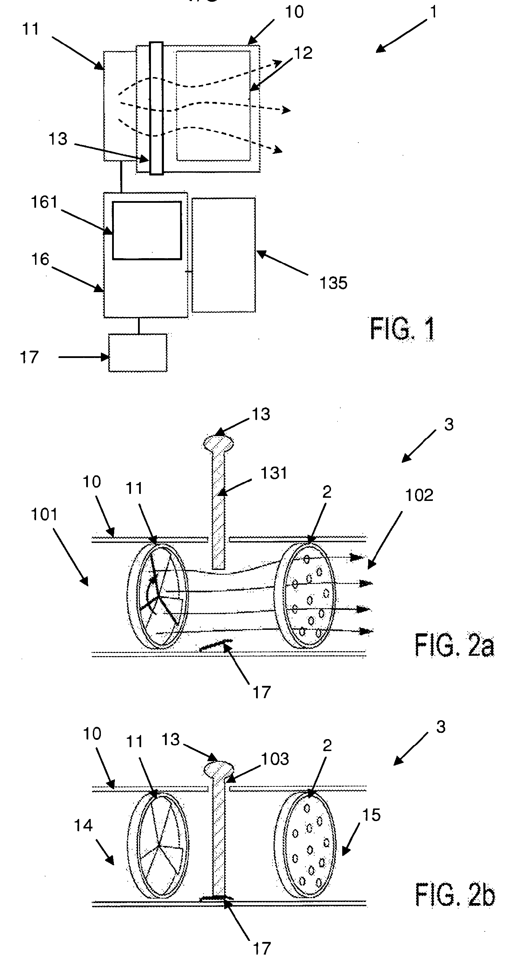

Comme illustré sur la figure 1, le dispositif 1 compact de diffusion de parfum comprend :
- un couloir de ventilation 10,
- un générateur 11 de flux d'air,
- un support de fixation 12,
- une cloison 13,
- un asservissement 16 entre le générateur de flux d'air et la cloison.

Ces composants du dispositif peuvent être agencés dans un boîtier.

Le couloir de ventilation 10 comprend une entrée 101 et une sortie 102. Il est ouvert par son entrée et sa sortie sur l'environnement extérieur. Sa fonction est de guider l'air selon une direction de ventilation définie entre son entrée et sa sortie. Sa section dans un plan perpendiculaire à la direction de ventilation peut être de la forme d'un rectangle, d'un cercle, etc. Le couloir de ventilation peut être formé par une partie du boîtier.

Le générateur 11 de flux d'air est agencé pour propulser de l'air dans le couloir de ventilation. Plus particulièrement, le générateur de flux d'air peut être :
- attaché, par exemple par un clips, à l'entrée du couloir de ventilation,
- placé dans le couloir de ventilation, ou
- maintenu par un premier organe de maintien du boîtier,
sa fonction consistant à propulser l'air depuis l'environnement extérieur, de sorte que l'air circule d'amont en aval depuis l'entrée jusqu'à la sortie du couloir de ventilation.

Le générateur 11 de flux d'air comprend par exemple un ventilateur. Un ventilateur bon marché est préféré de façon à minimiser le coût du dispositif. Cependant, ce type de ventilateur comprend souvent des pâles en plastique de qualité médiocre et notamment extrêmement poreux et attaquable par les parfums en ce sens que les molécules de parfum qui l'atteignent s'y imprègnent aisément. Le générateur de flux d'air comprend également une source en énergie électrique, par exemple une pile, ou mécanique, par exemple par un ressort remonté manuellement.

Le support de fixation 12 est agencé en aval du générateur de flux d'air et adapté pour y fixer une cartouche de parfum 2, de sorte que la cartouche de parfum se trouve sur le passage de l'air propulsé. Plus particulièrement, le support de fixation peut être :
- attaché, par exemple par un clips, à la sortie du couloir de ventilation,
- placé dans le couloir de ventilation, ou
- maintenu par un deuxième organe de maintien du boîtier.

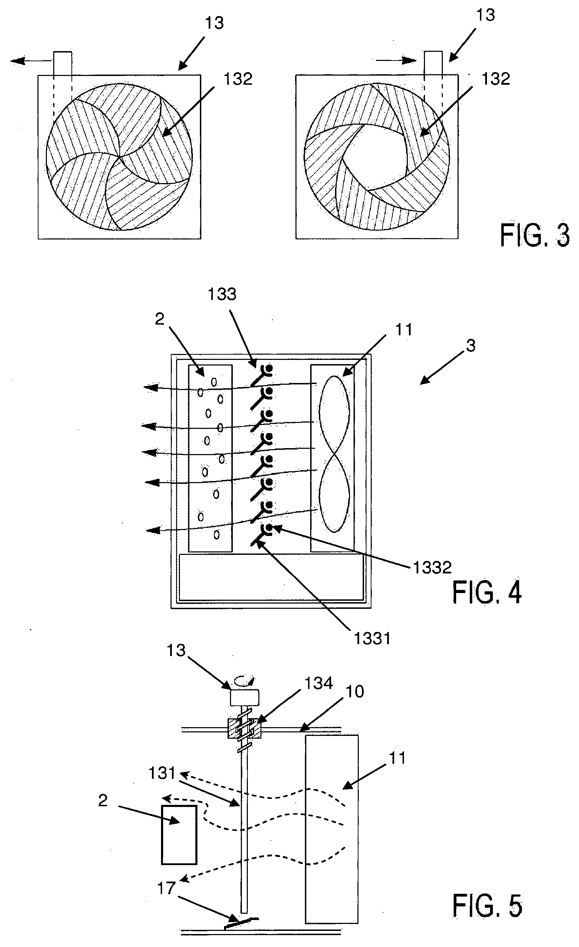

On entend par cartouche de parfum 2 une pièce qui laisse l'air passer à travers ou autour d'elle. La cartouche de parfum contient du parfum de sorte que des molécules de parfum soient emportées par l'air traversant la cartouche ou passant autour de la cartouche. Les dimensions de la cartouche de parfum sont proportionnées aux dimensions du couloir de ventilation. Selon un premier exemple illustrés sur les figures 2a et 2b, le pourtour extérieur de la cartouche est de dimensions égales au pourtour intérieur du couloir de ventilation de façon à pouvoir y être imbriquée et la cartouche laisse l'air passer au travers elle. Selon un deuxième exemple illustré sur la figure 5, le pourtour extérieur de la cartouche est de dimensions inférieures au pourtour intérieur du couloir de ventilation de façon à ce que la cartouche n'occupe qu'une partie de la section du couloir de ventilation et la cartouche laisse l'air passer autour d'elle.

La cloison 13 est située dans le couloir de ventilation 10 entre le générateur de flux d'air 11 et le support de fixation 12. L'état de la cloison est susceptible d'être modifié entre au moins :
- un état de cloisonnement dans lequel la cloison empêche l'air de circuler entre un premier espace 14 du couloir de ventilation en amont de la cloison et un second espace 15 du couloir de ventilation en aval de la cloison, et
- un état de non-cloisonnement dans lequel la cloison laisse l'air circuler entre les premier et second espaces du couloir de ventilation.

La cloison est réalisée en un matériau imperméable à l'air.

Dans l'état de cloisonnement, la cloison permet d'empêcher l'air de circuler. La jonction entre la cloison et le couloir de ventilation n'est pas nécessairement d'une herméticité absolue. Un jeu entre la cloison et le couloir de ventilation est autorisé qui est typiquement inférieur à 2 mm.

La section du conduit peut être constante ou varier. Par exemple, la section du couloir de ventilation en amont de la cloison est plus petite que la section du couloir de ventilation en aval de la cloison.

Les dimensions de la surface de la cloison sont proportionnées aux dimensions de la section du conduit de ventilation, et plus particulièrement aux dimensions de la section du conduit de ventilation en amont de la cloison. Dans un mode de réalisation, la surface de la cloison est supérieure à la section du couloir de ventilation selon au moins une de leurs dimensions, la cloison scindant en deux parties le couloir de ventilation et des organes de maintien (non représentés) étant agencés entre lesdites deux parties du couloir de ventilation pour maintenir la cloison au moins dans son état de cloisonnement. Dans un autre mode de réalisation, la surface de la cloison est légèrement inférieure à la section du couloir de ventilation selon au moins une de leurs dimensions, une différence typiquement inférieure à 2 mm selon au moins une dimension étant autorisée et un côté du pourtour intérieur du couloir de ventilation selon cette dimension jouant le rôle de butée pour maintenir la cloison au moins dans son état de cloisonnement.

Ainsi, l'air chargé en molécules de parfum ne peut pas être refoulé dans le premier espace vers le générateur de flux d'air, plus particulièrement vers les pâles du ventilateur ou tout autre composant comprenant des matériaux susceptibles de s'imprégner de parfums.

On entend par asservissement un système automatique dont le fonctionnement est régi par l'écart entre le comportement actuel et le comportement désiré. L'asservissement 16 entre le générateur de flux d'air et la cloison est adapté pour que la cloison soit en état de cloisonnement lorsque le générateur de flux d'air est à l'arrêt. L'asservissement permet d'une part de garantir le bon fonctionnement du dispositif, d'autre part de simplifier l'utilisation du dispositif par son automatisation.

La cloison et le couloir de ventilation sont réalisés au moins en partie en un matériau inerte aux parfums. On entend par un matériau inerte aux parfums un matériau avec lequel les molécules de parfum n'interagissent pas et donc qui ne peut pas être imprégné ou contaminé par les parfums. Ledit matériau inerte aux parfums peut être, de manière non limitative, de l'aluminium, un métal tel que le cuivre ou le zinc, du verre ou un matériau traité en surface pour empêcher les molécules de parfum de s'y imprégner.

Dans un mode de réalisation préféré, les parties du couloir de ventilation et de la cloison réalisées en un matériau inerte aux parfums sont constituées des surfaces internes limitant le second espace 15 du couloir de ventilation en aval de la cloison dans son état de cloisonnement. Ainsi, seule la face de la cloison orientée vers le support de fixation et le pourtour intérieur de la partie du couloir de ventilation en aval de la cloison sont en un matériau inerte aux parfums. Le coût du dispositif selon ce mode de réalisation est réduit par rapport au coût d'un dispositif réalisé entièrement en un matériau inerte aux parfums, les matériaux inertes aux parfums étant plus onéreux que les matériaux sensibles aux parfums généralement utilisés dans le domaine de l'invention, tels que les plastiques.

Ainsi, le dispositif 1 de diffusion de parfum présente plusieurs avantages.

Premièrement, il permet avantageusement de résoudre le problème de la pollution entre parfums en empêchant la diffusion des molécules olfactives depuis la cartouche de parfum 2 jusque dans ledit premier espace 14 du couloir de ventilation dans lequel se situe le générateur de flux d'air, et ce que le dispositif soit en fonctionnement ou à l'arrêt ; en fonctionnement, le ventilateur crée un flux d'air qui s'oppose à la diffusion des molécules olfactives dans ledit premier espace, et, à l'arrêt, la cloison empêche la diffusion des molécules olfactives dans ledit premier espace.

Deuxièmement, il présente une simplicité d'utilisation du fait de son automatisation qui évite à l'utilisateur d'avoir à manipuler le dispositif de sorte que ce dernier résolve le problème de la pollution entre parfums, ce problème devenant avantageusement transparent à l'utilisateur.

Troisièmement, il présente un faible coût. En effet, d'une part il ne nécessite pas un générateur de flux d'air comprenant un ventilateur aux pâles réalisées en un matériau inerte aux parfums qui serait coûteux, d'autre part, contrairement au produit ScentWave® décrit en introduction, le ventilateur et le couloir de ventilation ne sont pas des consommables au même titre que la cartouche de parfum, qui constitue le seul élément du dispositif selon l'invention à substituer à un autre pour changer le parfum diffusé ou recharger le dispositif en parfum.

Ces avantages font avantageusement du dispositif de diffusion de parfum selon l'invention un produit grand public.

Selon un premier mode de réalisation du dispositif, notamment représenté sur les figures 2a et 2b, la cloison 13 comprend un panneau 131 agencé pour coopérer avec une fente traversante 103 pratiquée dans le couloir de ventilation, de sorte que le panneau coulisse dans la fente. La fente est préférentiellement pratiquée perpendiculairement à la direction de circulation moyenne de l'air dans le couloir de ventilation. Le panneau est ainsi lui-aussi perpendiculaire à la direction de circulation moyenne de l'air dans le couloir de ventilation. Toutefois, un écart de quelques degrés, voire quelques dizaines de degrés, par rapport à cette perpendiculaire est envisageable sans s'éloigner de l'invention telle que revendiquée.

Dans ce premier mode de réalisation, la cloison est avantageusement de conception simple et peu onéreuse. La cloison peut également être amovible du couloir, voire du boîtier, et comporter un organe de préhension permettant à l'utilisateur de la retirer ou de l'insérer par coulissement dans la fente. La cloison peut ainsi avantageusement être changée par une nouvelle cloison et/ou aisément nettoyée, par exemple à l'alcool, pour enlever les molécules de parfum éventuellement retenues à sa surface inerte aux parfums exposée à la cartouche de parfum.

Selon une particularité non conforme à l'invention, le dispositif comprend en outre un capteur 17 agencé pour détecter au moins que la cloison quitte l'état de cloisonnement. L'asservissement consiste en une électronique de contrôle 161 propre à mettre en fonctionnement le générateur de flux d'air lorsque le capteur détecte que la cloison quitte l'état de cloisonnement. Par exemple, le capteur est un contacteur.

Le dispositif prévoit ainsi avantageusement la mise en fonctionnement automatique du générateur de flux d'air dès lors que la cloison n'est plus en son état de cloisonnement.

Selon une autre particularité non conforme à l'invention, le capteur est en outre agencé pour détecter que la cloison atteint l'état de cloisonnement. L'électronique de contrôle est alors en outre propre à arrêter le générateur de flux d'air lorsque le capteur détecte que la cloison atteint l'état de cloisonnement.

Le dispositif présente ainsi avantageusement une simplicité et un confort d'utilisation accrus et permet automatiquement de ne pas laisser inutilement le générateur de flux d'air en fonctionnement lorsque la cloison est en son état de cloisonnement. Le dispositif selon l'invention est ainsi avantageusement portable en ce sens qu'il ne nécessite pas d'être branché au réseau électrique, ses besoins en énergie étant faibles et autant que possible économes.

Par exemple lorsque l'utilisateur retire la cloison, le capteur le détecte et le ventilateur est mis automatiquement en fonctionnement, et, lorsque l'utilisateur insère la cloison, le capteur le détecte et le ventilateur est automatiquement arrêté.

Selon une variante du premier mode de réalisation du dispositif, le coulissement du panneau dans la fente est réalisé par renversements du dispositif. Le panneau coulisse alors sous l'action de son propre poids. Une butée agencée dans le boîtier permet de maintenir la cloison de sorte qu'un autre renversement du dispositif la ramène dans son état de cloisonnement. Par exemple, lorsque l'utilisateur renverse le dispositif, la cloison coulisse dans son état de non-cloisonnement, le capteur le détecte et le ventilateur est mis automatiquement en fonctionnement, et, lorsque l'utilisateur renverse à nouveau le dispositif, la cloison coulisse en son état de cloisonnement, le capteur le détecte et le ventilateur est automatiquement arrêté. Selon cette variante, le capteur peut aussi bien être un contacteur, comme précédemment envisagé, qu'un capteur gyroscopique.

Selon une autre variante du premier mode de réalisation du dispositif représentée sur la figure 5, ce dernier comprend une vis sans fin 134 agencée pour faire coulisser le panneau dans la fente. La vis sans fin est entraînée en rotation par un moteur 135, par exemple un moteur basse tension. Le panneau coulisse verticalement d'une position basse correspondant à l'état de cloisonnement à une position haute correspondant à l'état de non-cloisonnement. Le maintien d'une tension minimale aux bornes du moteur permet le maintien du panneau en position haute. L'ensemble formé du moteur et de la vis sans fin sont adaptés pour laisser retomber le panneau lorsque la tension devient inférieure à un certain seuil.

L'alimentation en énergie du moteur est avantageusement la même que celle du générateur de flux d'air.

Le dispositif selon cette autre variante du premier mode de réalisation prévoit ainsi avantageusement qu'en cas de panne d'alimentation en énergie du générateur de flux d'air, le panneau retombe en son état de cloisonnement. Les parties du dispositif se trouvant en amont de la cloison sont ainsi protégées de la pollution par les molécules olfactives même en cas de panne d'alimentation en énergie du dispositif.

Selon l'invention le dispositif ne comprend pas de capteur, mais comprend un interrupteur propre à mettre en fonctionnement et à arrêter le générateur de flux d'air. L'asservissement consiste alors en une électronique de contrôle propre à modifier l'état de la cloison. Par exemple, le générateur de flux d'air est mis en fonctionnement via l'interrupteur, puis l'électronique de contrôle modifie l'état de la cloison depuis son état de cloisonnement jusqu'à son état de non-cloisonnement ; ou inversement, l'électronique de contrôle modifie l'état de la cloison depuis son état de non-cloisonnement jusqu'à son état de cloisonnement avant que le générateur de flux d'air ne soit arrêté.

Le dispositif permet ainsi avantageusement de mettre automatiquement la cloison en son état de non-cloisonnement dès lors que le générateur de flux d'air est en fonctionnement et de mettre automatiquement la cloison en son état de cloisonnement dès lors que le générateur de flux d'air est arrêté. Une gestion plus fine du fonctionnement du dispositif est atteinte en ce sens que la diffusion de parfum dans le premier espace comportant le générateur de flux d'air est évitée que le générateur de flux d'air soit en fonctionnement, à l'arrêt ou dans un régime transitoire. On entend ici par régime transitoire du générateur de flux d'air la période de fonctionnement pendant laquelle le ventilateur est mis en tension, mais n'a pas encore atteint sa vitesse de rotation de fonctionnement ou la période de fonctionnement pendant laquelle le ventilateur est mis hors tension, mais où les pâles tournent encore, avant leur arrêt complet.

Dans un deuxième mode de réalisation, la cloison comprend un diaphragme 132 tel que représenté sur la figure 3. Le diaphragme est plus particulièrement un diaphragme à iris permettant une modification continue de l'état de la cloison entre son état de cloisonnement et son état de non-cloisonnement. Le dispositif permet ainsi avantageusement de faire varier le débit d'air atteignant la cartouche de parfum. Il est ainsi avantageusement permis à l'utilisateur de faire varier le pouvoir diffuseur du dispositif, par exemple en fonction du volume de la pièce dans lequel il diffuse ou pour économiser le parfum.

Le dispositif selon son deuxième mode de réalisation comprend :
- un interrupteur propre à mettre en fonctionnement et à arrêter le générateur de flux d'air, l'asservissement consistant en une électronique de contrôle propre à modifier l'état du diaphragme, de la façon décrite ci-dessus.

Dans un troisième mode de réalisation représenté sur la figure 4, la cloison comprend une persienne 133. La persienne comprend une ou plusieurs lames 1331. Chaque lame est montée mobile en rotation autour d'un axe horizontal 1332 de façon à se soulever sous la force de l'air propulsé par le générateur de flux d'air et à s'affaisser lorsque le générateur de flux d'air s'arrête. En cas de pluralité de lames, chaque lame en position affaissée repose sur la lame du dessous, la lame la plus basse reposant sur le fond du couloir, de sorte que les lames affaissées constituent la cloison en état de cloisonnement.

Le dispositif permet ainsi avantageusement d'asservir la modification de l'état de cloisonnement de la cloison au fonctionnement du générateur de flux d'air uniquement de façon mécanique. L'asservissement est réalisé par la seule force de l'air propulsé par le ventilateur. Plus particulièrement, le dispositif dans son troisième mode de réalisation non conforme à l'invention ne requière ni une électronique de contrôle, ni un capteur, seul un interrupteur restant nécessaire pour mettre en fonctionnement ou arrêter le ventilateur.

Selon une autre particularité d'un quelconque mode de réalisation du dispositif, ce dernier comprend une seconde cloison agencée en aval du support de fixation.

L'état de la seconde cloison est susceptible d'être modifié entre au moins :
- un état de cloisonnement dans lequel la seconde cloison empêche l'air de circuler entre le second espace 15 du couloir de ventilation en aval de la cloison et l'environnement extérieur, et
- un état de non-cloisonnement dans lequel la cloison laisse l'air circuler entre le second espace 15 du couloir de ventilation et l'environnement extérieur.

Le dispositif permet ainsi avantageusement d'éviter une diffusion rémanente des molécules de parfum vers l'extérieur du dispositif, lorsque le dispositif est à l'arrêt.

La modification d'état de la seconde cloison peut être manuelle. Dans le cas où le support de fixation est placé dans le couloir de ventilation, la seconde cloison peut être attachée de façon amovible, par exemple par un clips, à la sortie du couloir de ventilation. Il fonctionne alors à la façon d'un simple opercule d'objectif d'appareil photographique. Tout comme dans cet exemple illustratif, il est de la responsabilité de l'utilisateur de l'enlever avant de mettre le ventilateur en fonctionnement, car il est à éviter une situation dans laquelle le ventilateur serait en fonctionnement, la première cloison 13 en position de non-cloisonnement et la seconde cloison en position de cloisonnement. Cette situation conduirait en effet à refouler de l'air chargé en molécules olfactives vers le premier espace 14 du couloir de ventilation en amont de la première cloison.

Il est possible de garantir de façon automatique la non-occurrence de cette situation si la modification d'état de la seconde cloison est asservie comme l'autre au fonctionnement du générateur de flux d'air, ou directement à la modification d'état de la première cloison 13.

La seconde cloison ou la face de la seconde cloison orientée vers le support de fixation est réalisée en un matériau inerte aux parfums.

Il est à noter que la cartouche de parfum est par nature jetable et remplacée après usage. A cette fin, le dispositif 1 de diffusion de parfum comprend des moyens d'insertion et de retrait ou d'éjection (moyens non représentés) d'une cartouche dans le couloir de ventilation 10 au niveau du support de fixation 12. Ces moyens d'insertion et de retrait sont agencés conjointement ou non avec le support de fixation 12. Il peut s'agir par exemple d'un mécanisme dit « push-to-open » fonctionnant à la manière des mécanismes d'insertion et de retrait des cartes mémoires dans les appareils photos : on insère la cartouche dans le dispositif et on appuie dessus pour verrouiller le mécanisme, puis on réappuie dessus pour déverrouiller le mécanisme et pouvoir retirer la cartouche. Selon, un autre exemple, les moyens d'insertion et de retrait consistent en un mécanisme de translation verticale, avec un plateau et un crochet de verrouillage, pour descendre et relever la cartouche 2 dans et depuis le dispositif 1 respectivement, comme dans un grille-pain.

Par ailleurs, certaines caractéristiques techniques de plusieurs autres modes de réalisation du présent dispositif compact de diffusion de parfum sont étroitement liées aux caractéristiques techniques d'une cartouche de parfum particulière qui va être brièvement décrite ci-dessous, en préambule de la description desdits autres modes de réalisation du présent dispositif.

Ladite cartouche de parfum particulière comprend essentiellement :
- des éléments de substrat dans lesquels est adsorbé un parfum,
- un cadre,
- une première grille et une seconde grille fixées dans le cadre en vis-à-vis l'une de l'autre, chacune des grilles comprenant une pluralité de barreaux.

Par exemple, les éléments de substrat sont des billes sphéroïdes de matériau polymère et les molécules de parfum sont adsorbées dans tout le volume de chaque bille.

Les billes en matériau polymère présentent chacune, avant adsorption du parfum, une plus petite dimension égale à 3 mm et une plus grande dimension égale à 4 mm, et, après adsorption du parfum, une plus petite dimension égale à 4 mm et une plus grande dimension égale à 6 mm.

Le flux d'air traversant la cartouche induit un accroissement de la quantité de molécules de parfum diffusées par unité de temps, par rapport à la quantité de molécules de parfum qui seraient évaporées par unité de temps en l'absence de flux d'air. Le phénomène de désorption se conjugue effectivement avec la convection du flux d'air.

Selon une particularité de la cartouche, les barreaux d'une même grille sont espacés entre eux et les grilles sont espacées entre elles, de sorte que la cartouche permette le maintien dans le cadre et entre les grilles d'une seule couche d'éléments de substrat.

Les éléments de substrat sont ainsi disposés dans la cartouche sur une seule couche, ce qui présente plusieurs avantages.

Tout d'abord, une diffusion satisfaisante des molécules de parfum dans l'atmosphère par le flux d'air nécessite une pression moindre que celle qui serait requise si des éléments de substrat étaient disposés sur plusieurs couches. Les contraintes auxquelles doit satisfaire le générateur 11 de flux d'air en sont d'autant moins élevées. Typiquement, l'utilisation de ventilateurs ou de turbines de faibles ou très faibles puissances, et donc bons marchés et relativement silencieux, est avantageusement suffisante et satisfaisante. Typiquement, une surpression de l'air traversant la cartouche de l'ordre de 0,10 à 0,80 inch H2O à 21°C, soit de l'ordre de, 25 à 200 pascals, offre un résultat très satisfaisant. Il est à noter que, dans le cas où plusieurs cartouches sont juxtaposées entre elles tel que vu plus haut, la puissance de la turbine doit proportionnellement être augmentée dans la fourchette susmentionnée. Dans le cas où une seule cartouche est disposée dans le dispositif, il sera préféré une turbine créant une surpression de l'air traversant la cartouche comprise entre l'ordre de 25 et 50 pascals.

Selon une autre caractéristique technique de la cartouche 2, elle présente une largeur et une longueur/hauteur de l'ordre de quelques centimètres, et préférentiellement égales à 4,7 cm, et une épaisseur de l'ordre du centimètre, et préférentiellement égale à 1,4 cm.

Ainsi, les dimensions de la cartouche, ou équivalemment de son cadre, sont relativement faibles, ce qui est compatible avec des dispositifs 1 compacts. Et, dans un mode de réalisation du dispositif 1, le couloir de ventilation 10 présente une section transverse de largeur et de longueur/hauteur sensiblement plus élevées que celles de la cartouche, de façon à ce que la cartouche puisse être insérée sensiblement sans jeu dans le couloir de ventilation. Typiquement, le couloir de ventilation présente un pourtour intérieur ayant une section transverse de largeur et de longueur/hauteur égales à 4,8 cm. Ainsi, aucun espace n'est laissé entre le cadre de la cartouche 2 et le couloir de ventilation 10, et tout l'air propulsé traverse la cartouche.

Dans un de ses modes de réalisation, le dispositif 1 compact de diffusion de parfum se présente sous la forme d'un parallélépipède de :
- moins de 20 cm de large, préférentiellement 5,3 cm de large,
- plus de 2 cm et moins de 15 cm de profondeur, et
- moins de 20 cm de haut, préférentiellement 11,5 cm de haut.

Dans ce mode de réalisation, le couloir de ventilation 10 est agencé dans la profondeur du dispositif de sorte que l'air soit aspiré depuis le dos du dispositif et propulsé vers l'avant de celui-ci. En outre, le couloir de ventilation 10 court sur toute la profondeur du dispositif et s'étend sur toute la largeur du dispositif et sur une distance comprise entre le tiers et la moitié de la hauteur totale du dispositif.

Par ailleurs, le cadre de la cartouche 2 peut comprendre au moins un élément mâle de fixation sur une face et au moins autant d'éléments femelles de fixation sur l'autre face, chaque élément mâle étant propre à mettre en prise, de façon préférentiellement amovible, l'un quelconque des éléments femelles. De cette façon, plusieurs cartouches peuvent être jointes ensemble, leurs cadres se juxtaposant dans la direction de leur épaisseur.

C'est dans cette option qu'est prévu ci-dessus que la profondeur du parallélépipède formant le dispositif peut varier entre 2 et 20 cm, car en effet si, lorsqu'une seule cartouche est chargeable dans le dispositif, celui présente préférentiellement une profondeur inférieure à 5 cm, plusieurs cartouches peuvent être juxtaposées dans leur épaisseur augmentant d'autant la profondeur requise du dispositif. Cette possibilité de juxtaposer les cartouches entre elles reste compatible avec un dispositif de diffusion de parfum compact, du fait de la faible épaisseur des cartouches.

En outre, le dispositif comprend dans un autre mode de réalisation un dispositif de détection de la présence ou de l'absence d'au moins une cartouche dans le couloir de ventilation, de sorte que ce dispositif conjointement avec l'électronique de contrôle du dispositif permette d'éviter la mise en fonctionnement du générateur de flux d'air en cas d'absence d'au moins une cartouche dans le couloir de ventilation. Par exemple, ledit dispositif de détection de la présence ou de l'absence d'au moins une cartouche dans le couloir de ventilation peut avantageusement reposer sur une coopération mécanique d'un des éléments mâles, prévus pour la clipsabilité des cartouches entre elles, avec un interrupteur de courant d'alimentation du générateur de flux d'air. L'introduction d'une cartouche 2 dans le support de fixation 12 du dispositif 1 permet d'exercer par contact dudit élément mâle une pression sur l'interrupteur de sorte que ce dernier passe en une position fermée du circuit d'alimentation du générateur de flux d'air, lorsque la cartouche est complètement et correctement insérée dans le dispositif. Cela permet notamment d'économiser de l'énergie électrique en interdisant le fonctionnement du dispositif lorsqu'il n'y a pas de cartouche dans le dispositif.

D'autres caractéristiques techniques de la cartouche, sont avantageusement exploitées.

Il s'agit tout d'abord du fait que chaque élément de substrat est réalisé en un matériau polymère dans lequel est adsorbé du parfum et donc présente des dimensions qui varient dans le temps depuis des dimensions initiales jusqu'à des dimensions finales plus petites atteintes lorsque le parfum est entièrement désorbé. Les dimensions initiales des éléments de substrat sont généralement comprises entre 3 mm et 8 mm. Plus particulièrement, la plus petite dimension initiale des billes en matériau polymère est égale à 4 mm et la plus grande dimension initiale des billes en matériau polymère est égale à 6 mm, ce qui correspond exactement aux dimensions indiquées plus haut des billes en matériau polymère après adsorption du parfum. Les dimensions finales des éléments de substrat 1 en matériau polymère sont comprises entre un demi et huit dixièmes de leurs dimensions initiales. Plus particulièrement, la plus petite dimension finale des billes en matériau polymère est égale à 3 mm et la plus grande dimension finale des billes en matériau polymère est égale à 4 mm, ce qui correspond exactement aux dimensions indiquées plus haut des billes en matériau polymère avant adsorption du parfum.

Il s'agit ensuite du fait que deux barreaux seconds voisins de la première grille s'étendent dans l'épaisseur du cadre, de sorte que, entre ces deux barreaux seconds voisins disposés verticalement, une seule rangée d'éléments de substrat dont le nombre est maîtrisé puisse être maintenue.

Dès lors, les éléments de substrat de la rangée devenant dans le temps libres en mouvement vertical entre lesdits barreaux seconds voisins et se tassant au moins sous l'action de leur poids, la hauteur de la rangée constitue un indicateur visuel d'usure de la cartouche.

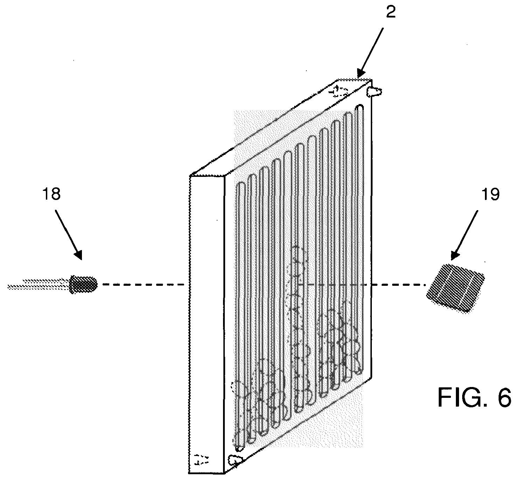

Ainsi, le dispositif 1 comprenant en outre, dans un nouveau mode de réalisation dont le principe est illustré sur la figure 6, une source de lumière 18, telle qu'une diode électroluminescente, et un capteur d'intensité lumineuse 19, tel qu'une cellule photovoltaïque, situés de part et d'autre de la rangée et à une hauteur déterminée permet de capter le passage sous une hauteur seuil de la rangée, et ainsi avertir l'utilisateur de l'usure de la cartouche et son nécessaire remplacement. Cet avertissement peut plus particulièrement consister en un signal lumineux émanant d'une deuxième diode électroluminescente agencée sur une façade du dispositif et alimentée par la cellule photovoltaïque.

La présente invention concerne également un système 3 de diffusion de parfum. Le système comprend un dispositif 1 de diffusion de parfum tel que décrit ci-dessus et une cartouche de parfum 2 adaptée pour être fixée dans le support de fixation 12.

Le système présente ainsi avantageusement un faible coût, au moins du fait que le générateur de flux d'air et le couloir de ventilation ne sont pas des consommables au même titre que la cartouche de parfum, cette dernière étant le seul élément du système à devoir être remplacé pour changer le parfum diffusé ou recharger le dispositif en parfum.

Notamment avant de changer de parfum à diffuser, les molécules olfactives éventuellement déposées sur les surfaces internes limitant le second espace 15 du couloir de ventilation en aval de la cloison dans son état de cloisonnement sont avantageusement arrachées à ces surfaces et dispersées dans l'environnement extérieur en mettant en fonctionnement le générateur de flux d'air, lorsque la(les) cloison(s) sont en état de non cloisonnement et en l'absence de cartouche de parfum dans le support de fixation.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés, et notamment combinés, dans le domaine défini par la portée des revendications jointes.

## Revendications

1. Dispositif (1) compact de diffusion de parfum comprenant :
- un couloir de ventilation (10) comprenant une entrée (101) et une sortie (102), et
- un générateur (11) de flux d'air agencé pour propulser de l'air dans le couloir de ventilation, de sorte que l'air circule d'amont en aval depuis l'entrée jusqu'à la sortie du couloir de ventilation,
- un support de fixation (12) agencé en aval du générateur de flux d'air et adapté pour y fixer une cartouche de parfum (2), de sorte que la cartouche de parfum se trouve sur le passage de l'air propulsé,
- une cloison (13) située dans le couloir de ventilation entre le générateur de flux d'air et le support de fixation, l'état de la cloison étant susceptible d'être modifié entre au moins :
o un état de cloisonnement dans lequel la cloison empêche l'air de circuler entre un premier espace (14) du couloir de ventilation en amont de la cloison et un second espace (15) du couloir de ventilation en aval de la cloison, et
o un état de non-cloisonnement dans lequel la cloison laisse l'air circuler entre les premier et second espaces du couloir de ventilation, et
- un asservissement (16) entre le générateur de flux d'air et la cloison adapté pour que la cloison soit en état de cloisonnement lorsque le générateur de flux d'air est à l'arrêt,
la cloison et le couloir de ventilation étant réalisés au moins en partie en un matériau inerte aux parfums qui ne s'imprègne pas de parfums,
**caractérisé en ce qu'**il comprend un interrupteur propre à mettre en fonctionnement et à arrêter le générateur (11) de flux d'air, l'asservissement consistant en une électronique de contrôle (161) propre à modifier l'état de la cloison (13) de sorte que :
- la cloison quitte l'état de cloisonnement après que le générateur de flux d'air ait été mis en fonctionnement, et
- la cloison atteint l'état de cloisonnement avant que le générateur de flux d'air soit à l'arrêt.

2. Dispositif (1) selon la revendication 1, **caractérisé en ce que** les parties du couloir de ventilation (10) et de la cloison (13) réalisées en un matériau inerte aux parfums sont constituées des surfaces internes limitant le second espace (15) du couloir de ventilation en aval de la cloison dans son état de cloisonnement.

3. Dispositif (1) selon l'une quelconque des revendications précédentes, **caractérisé en ce que** la cloison (13) comprend un panneau (131) agencé pour coopérer avec une fente traversante (103) pratiquée dans le couloir de ventilation (10), de sorte que le panneau coulisse dans la fente.

4. Dispositif (1) selon la revendication 3, **caractérisé en ce que** le coulissement du panneau (131) dans la fente traversante (103) est réalisé par renversements du dispositif, le panneau coulissant sous l'action de son propre poids.

5. Dispositif (1) selon la revendication 3, **caractérisé en ce qu'**il comprend une vis sans fin (134) agencée pour faire coulisser le panneau (131) dans la fente traversante (103), la vis sans fin (134) étant entraînée en rotation par un moteur (135), le panneau (131) coulissant verticalement d'une position basse correspondant à l'état de cloisonnement à une position haute correspondant à l'état de non-cloisonnement, une tension minimale aux bornes du moteur (135) permettant le maintien du panneau en position haute, et, l'ensemble formé du moteur et de la vis sans fin étant adapté pour laisser retomber le panneau lorsque la tension devient inférieure à un certain seuil.

6. Dispositif (1) selon l'une des revendications 1 à 2, **caractérisé en ce que** la cloison (13) comprend un diaphragme (132).

7. Dispositif (1) selon l'une des revendications 1 et 2, **caractérisé en ce que** la cloison (13) comprend une persienne (133) comprenant une pluralité de lames (1331), chaque lame étant montée mobile en rotation autour d'un axe horizontal (1332) de façon à se soulever sous la force de l'air propulsé par le générateur de flux d'air et à s'affaisser lorsque le générateur (11) de flux d'air s'arrête.

8. Dispositif (1) selon l'une quelconque des revendications précédentes, **caractérisé en ce qu'**il comprend une seconde cloison agencée en aval du support de fixation.

9. Dispositif (1) selon l'une quelconque des revendications précédentes, **caractérisé en ce qu'**il comprend des moyens d'insertion et de retrait d'au moins une cartouche (2) dans le couloir de ventilation (10) au niveau du support de fixation (12).

10. Dispositif (1) selon l'une quelconque des revendications précédentes, **caractérisé en ce que** le générateur (11) de flux d'air comprend une turbine générant une surpression de l'air traversant la cartouche qui est comprise entre 25 et 200 pascals.

11. Dispositif (1) selon l'une quelconque des revendications précédentes, **caractérisé en ce qu'**il se présente sous la forme générale d'un parallélépipède de :
- moins de 20 cm de large, préférentiellement 5,3 cm de large,
- plus de 2 cm et moins de 20 cm de profondeur, préférentiellement inférieure à 5 cm, et
- moins de 20 cm de haut, préférentiellement 11,5 cm de haut.

12. Dispositif (1) selon l'une quelconque des revendications 3 à 11, **caractérisé en ce qu'**il comprend en outre un dispositif de détection de l'absence de cartouche dans le couloir de ventilation, ce dispositif conjointement avec l'électronique de contrôle (161) permettant d'éviter la mise en fonctionnement du générateur de flux d'air en cas d'absence d'au moins une cartouche dans le couloir de ventilation.

13. Dispositif (1) selon l'une quelconque des revendications précédentes, **caractérisé en ce qu'**il comprend en outre une source de lumière (18) et un capteur d'intensité lumineuse (19) situés de part et d'autre du support de fixation et agencés de sorte de capter un état d'usure de la cartouche et en avertir l'utilisateur.

14. Système (3) de diffusion de parfum **caractérisé en ce qu'**il comprend un dispositif (1) selon l'une des revendications 1 à 13 et une cartouche de parfum (2) adaptée pour être fixée dans le support de fixation (12).

## Patentansprüche

1. Kompakte Duftspendervorrichtung, die folgendes umfasst:
- einen Luftdurchgang (10), welcher einen Eintritt (101) und einen Austritt (1°2) enthält, und
- einen Luftstromerzeuger (11), der so angeordnet ist, dass die Luft in den Luftdurchgang geleitet wird, und die Luft somit in einer von stromaufwärts nach stromabwärts gerichteten Strömung vom Eingang bis zum Ausgang des Luftdurchgangs zirkuliert,
- eine Montagehalterung (12) die unterhalb des Luftstromerzeugers angeordnet ist und so angepasst ist, dass eine Duftkassette (2) daran befestigt werden kann, so dass sich die Duftkassette im Durchgang des Luftstroms befindet,
- eine Trennwand (13) im Luftdurchgang zwischen dem Luftstromerzeuger und der Montagehalterung, wobei sich der Zustand der Trennwand zu mindestens folgenden Zuständen verändern kann:
o ein Abschottungszustand, in dem die Trennwand die Luft daran hindert, zwischen einem ersten Raum (14) des Luftdurchgangs oberhalb der Trennwand und einem zweiten Raum (15) des Luftdurchgangs unterhalb der Trennwand zu zirkulieren, und
- einen Zustand der Nichtabschottung, in dem die Trennwand die Luft zwischen dem ersten und dem zweiten Raum des Luftdurchgangs zirkulieren lässt, und
- eine Steuerung (16) zwischen dem Luftstromerzeuger und der Trennwand, die so angepasst ist, dass die Trennwand geschlossen ist, wenn der Luftstromerzeuger ausgeschaltet ist,
- wobei die Trennwand und der Luftdurchgang mindestens zum Teil aus einem Material gebildet werden, das inert für Duftstoffe ist und diese nicht annimmt,
**dadurch gekennzeichnet, dass** sie einen Schalter enthält, an welchem den Luftstromgenerator (11) ein- und ausgeschaltet werden kann, wobei die Steuerung aus einem elektronischen Kontrollsystem (161) besteht, das in der Lage ist, den Zustand der Trennwand (13) so zu verändern, dass:
- die Trennwand den Abschottungszustand aufhebt, nachdem der Luftstromerzeuger eingeschaltet wurde, und
- die Trennwand den Abschottungszustand herstellt, bevor der Luftstromerzeuger ausgeschaltet wird.

2. Vorrichtung (1) nach Anspruch 1, **dadurch gekennzeichnet, dass** die Teile des Luftdurchgangs (10) und der Trennwand (13), die aus einem für Duftstoffe inerten Material bestehen, Innenflächen bilden, die den zweiten Raum (15) des Luftdurchgangs unterhalb der Trennwand im abgeschotteten Zustand abgrenzen.

3. Vorrichtung (1) nach einem der vorhergehenden Ansprüche, **dadurch gekennzeichnet, dass** die Trennwand (13) eine Platte (131) umfasst, die so angeordnet ist, dass sie mit einem durchgehenden Schlitz (103) im Luftdurchgang (10) zusammenwirkt, so dass das Platte im Schlitz entlang gleitet.

4. Vorrichtung (1) nach Anspruch 3, **dadurch gekennzeichnet, dass** das Gleiten der Platte (131) im durchgehenden Schlitz (103) durch Umdrehen der Vorrichtung zustande kommt, wobei die Platte durch Einwirkung ihres eigenen Gewichts gleitet.

5. Vorrichtung (1) nach Anspruch 3, **dadurch gekennzeichnet, dass** sie eine Schnecke (134) enthält, die so angeordnet ist, dass die Platte (131) im durchgehenden Schlitz (103) entlang gleitet, wobei die Schnecke (134) durch einen Motor (135) rotierend angetrieben wird und die Platte (131) von einer unteren Stellung aus, welche dem Zustand der Abschottung entspricht, senkrecht zu einer oberen Stellung, welche dem Zustand der Nichtabschottung entspricht, gleitet, und eine Mindestspannung an den Klemmen des Motors (135) dafür sorgt, dass die Platte in der oberen Stellung gehalten wird, und die Einheit bestehend aus Motor und Schnecke so angepasst ist, dass die Platte herunter fällt, wenn die Spannung unter einen bestimmten Schwellenwert fällt.

6. Vorrichtung (1) nach einem der Ansprüche 1 bis 2, **dadurch gekennzeichnet, dass** die Trennwand (13) eine Membran(132) umfasst.

7. Vorrichtung nach einem der Ansprüche 1 und 2, **dadurch gekennzeichnet, dass** die Trennwand (13) eine Abdeckung (133) mit vielen Lamellen (1331) umfasst, wobei jede Lamelle drehbar um eine horizontale Achse (1332) montiert ist, so dass sie sich unter der einwirkenden Kraft des vom Luftstromgenerator erzeugten Luftstroms anhebt und sich absenkt, wenn der Luftstromgenerator (11) abgeschaltet sind.

8. Vorrichtung (1) nach einem der vorhergehenden Ansprüche, **dadurch gekennzeichnet, dass** sie eine zweite Trennwand enthält, die unterhalb der Befestigungshalterung angeordnet ist.

9. Vorrichtung (1) nach einem der vorhergehenden Ansprüche, **dadurch gekennzeichnet, dass** sie in Höhe der Montagehalterung (12) Mittel zum Einschieben und Rückholen von mindestens einer Kassette (2) in bzw. aus dem Luftdurchgang (10) enthält.

10. Vorrichtung (1) nach einem der vorhergehenden Ansprüche, **dadurch gekennzeichnet, dass** der Luftstromerzeuger (11) eine Turbine enthält, welche einen Luftüberdruck generiert, der die Kassette durchströmt und bei 25 bis 200 Pascal liegt.

11. Vorrichtung (1) nach einem der vorhergehenden Ansprüche, **dadurch gekennzeichnet, dass** sie generell die Form eines Quaders aufweist:
- mit einer Breite von mindestens 20 cm, vorzugsweise aber 5,3 cm
- einer Tiefe von über 2 cm und unter 20 cm, vorzugsweise aber kleiner als 5 cm, und
- eine Höhe von mindestens 20 cm, vorzugsweise aber 11,5 cm.

12. Vorrichtung (1) nach einem der Ansprüche 3 bis 11, **dadurch gekennzeichnet, dass** sie des weiteren eine Vorrichtung zur Erkennung des Fehlens einer Kassette im Luftdurchgang umfasst, wobei diese Vorrichtung zusammen mit dem elektronischen Steuersystem (161) das Einschalten des Luftstromerzeugers verhindert, wenn mindestens eine Kassette im Luftdurchgang fehlt.

13. Vorrichtung (1) nach einem der vorhergehenden Ansprüche, **dadurch gekennzeichnet, dass** sie des weiteren eine Lichtquelle (18) und einen Lichstärkesensor (19) zu beiden Seiten der Befestigungshalterung umfasst, die so angeordnet sind, dass sie einen Verschleißzustand der Kassette erkennen und den Benutzer warnen können.

14. Duftspendersystem (3) **dadurch gekennzeichnet, dass** es eine Vorrichtung (1) nach einem der Ansprüche 1 bis 13 enthält und eine Duftkassette (2), die so angepasst ist, dass sie an der Montagehalterung (12) befestigt werden kann.

## Claims

1. A compact device (1) for the diffusion of fragrance comprising:
- a ventilation duct (10) comprising an inlet (101) and an outlet (102), and
- an air flow generator (11), so arranged as to blow air into the ventilation duct, so that air flows in the downstream direction from the inlet to the outlet of the ventilation duct,
- a mounting bracket (12) which is arranged downstream of the air flow generator and so designed as to have a fragrance cartridge (2) fixed thereon, so that the fragrance is in the way of the air blown,
- a partition (13) located in the ventilation duct between the air flow generator and the mounting bracket, with the condition of the partition being liable of being changed between at least:
o a partitioning state and a non-partitioning state, in which the partition prevents air from flowing between a first space (14) of the ventilation duct upstream of the partition and a second space (15) of the ventilation duct downstream of the partition, and
o a non-partitioning condition in which the partition lets air flow between the first and second spaces of the ventilation duct, and
- a servo-control (16), between the air flow generator and the partition, so designed that the partition is in the partitioning state when the air flow generator is stopped,
with the partition and the ventilation duct being made at least in part of a material inert to fragrances, which will not be permeated with a fragrance,
**characterized in that** it comprises a switch able to start or to stop the air flow generator (11), with the servo-control consisting in control electronics (161) able to modify the condition of the partition (13) so that:
- the partition leaves the partitioning state when the air flow generator is started, and
- the partition reaches the partitioning state before the air flow generator is stopped.

2. A device (1) according to claim 1, **characterized in that** the parts of the ventilation duct (10) and of the partition (13) made of a material inert to fragrances comprise internal surfaces limiting the second space (15) of the ventilation duct downstream of the partition in its partitioning state.

3. A device (1) according to any one of the preceding claims, **characterized in that** the partition (13) comprises a panel (131) so arranged as to cooperate with a through slot (103) provided in the ventilation duct (10), so that the panel can slide in the slot.

4. A device (1) according to claim 3, **characterized in that** the sliding of the panel (131) in the through slot (103) is achieved by turning the device upside down, with the panel sliding under its own weight.

5. A device (1) according to claim 3, **characterized in that** it comprises a worm screw (134) so arranged as to make the panel (131) slide in the through slot (103), with the worm screw (134) being rotated by a motor (135), with the panel (131) vertically sliding from a lower position corresponding to the partitioning state to a higher position corresponding to the non-partitioning state, with a minimum voltage at the terminals of the motor (135) making it possible to hold the panel in the higher position, and with the assembly formed by the motor and the worm screw being adapted to let the panel fall back when the voltage drops below a certain threshold.

6. A device (1) according to one of claims 1 to 2, **characterized in that** the partition (13) comprises a diaphragm (132).

7. A device (1) according to one of claims 1 and 2, **characterized in that** the partition (13) comprises a shutter (133) comprising a plurality of blades (1331), with each blade being so mounted as to rotate about a horizontal axis (1332) so as to rise under the force of the air blown by the air flow generator and to fall back when the air flow generator (11) stops.

8. A device (1) according to any one of the preceding claims, **characterized in that** it comprises a second partition arranged downstream of the mounting bracket.

9. A device (1) according to any one of the preceding claims, **characterized in that** it comprises means for inserting and removing at least one cartridge (2) in/from the ventilation duct (10) at the mounting bracket (12).

10. A device (1) according to any one of the preceding claims, **characterized in that** the air flow generator (11) comprises a turbine generating an overpressure of the air flowing through the cartridge which ranges from 25 to 200Pa.

11. A device (1) according to any one of the preceding claims, **characterized in that** it generally has the shape of a parallelepiped:
- less than 20cm wide, preferably 5.3cm wide,
- more than 2cm and less than 20cm deep, preferably less than 5cm, and
- less than 20cm high, preferably 11.5cm high.

12. A device (1) according to any one of claims 3 to 11, **characterized in that** it further comprises a device for detecting the absence of a cartridge in the ventilation duct, with such device, together with the control electronics (161), making it possible to prevent the starting of the air flow generator in the absence of at least one cartridge in the ventilation duct.

13. A device (1) according to any one of the preceding claims, **characterized in that** it further comprises a light source (18) and a light intensity sensor (19) located on either side of the mounting bracket and so arranged as to sense a wear condition of the cartridge and notify the user thereof.

14. A system (3) for diffusing a fragrance **characterized in that** it comprises a device (1) according to one of claims 1 to 13 and a fragrance cartridge (2) adapted to be secured in the mounting bracket (12).
